# EUROPEAN PATENT APPLICATION

(11) **EP 1 254 639 A1**
(43) Date of publication of application: **06.11.2002**
(21) Application number: 00901620.5
(22) Date of filing: 10.02.2000
(51) Int. Cl.: A61B 17/70, A61B 17/86

(54) **DORSOLUMBAR AND LUMBOSACRAL VERTEBRAE FIXING SYSTEM**

(71) Applicant: INDUSTRIAS QUIRURGICAS DE LEVANTE, S.L., 46988 Paterna (ES)
(72) Inventor: BARBERA ALACREU, José, 46988 Paterna (ES)
(74) Representative: Koepe, Gerd L., Dipl.-Chem.
(86) International application number: ES0000041
(87) International publication number: WO01058369

(57) **Abstract**

The invention concerns a dorsolumbar and lumbosacral vertebrae fixing system which is based on connectors (1) in the form of a clamp (12) that is provided with a hollow multidirectional swivel joint (3) which is traversed by a rod (2). The clamp also has a circular or elliptical hole (13) through which the end (4) of the fixing element to the vertebra is fixed. Said element may be a hollow expansion screw (6) that opens against the spongy element when a pin (18) is inserted inside or when a hook (5) that is coupled to the venebra in the laminar or pedicular area in inserted. The invention can be used in the surgical implant industry.

## Description

The dorsolumbar and lumbosacral fixation system that is the object of this present invention consists of an assembly of connectable parts that form a solid and compact system consisting of vertebrae attachment elements that, by means of clamps that are adjustable around a swivel coupled to a rod, can be positioned so that by closing them, using locknuts, the desired fixation can be achieved.

To date different elements have been used as integral parts of vertebral fixation systems. One of these depends on connectors or couplings which always present a fixation screw at 90° to a rod, in such a way that the angle formed by the coupling axes for the pass of these two parts is straight.

The main disadvantage is that the onset between the screw and the rod is not always at an angle of 90°, as a result of which it is necessary to bend and turn the rod in order to bring the coupling to the screw head, increasing weak points, surgical intervention times, complications etc..

At the same time these connectors have a single orifice, i.e. there is no possibility of modifying the position of the rod when there is any difficulty of alignment of the rod in relation to the screw heads, and therefore it is necessary to bend the rod with the problems that are then involved in such operations.

Another of the elements used in current fixation systems, are rods that are fully threaded, in such a way that the connector has two locknuts for fixation, as well as setscrews and nut locking. At the same time, once the threaded rod has been moulded, there are problems in fitting the other parts into place, as well as the fact that the threads become stress-raisers that can lead to breakage and cracking through material fatigue. This also leads to plastic mis-shaping.

In short, this system leads to a great deal of mechanical complications, due to the number of parts involved, in such a way that the surgical intervention tends to be lengthened, increasing the risks of complications due to dropping or loss of parts, etc..

The pedicle screws that are used are self-threading, i.e., they tap into the vertebral pedicles, with threads that have been conditioned to this end. These screws usually encounter problems of rejection by the bone (pull-out effect), in that the thread worked into the bone, due to the constant movement thereof, can widen the hole which will result in a full or partial pull-out. Also, in cases of osteoporosis, there is no solid bone mass and the thread does not bind to the bone.

In cases of revision, when it may be necessary to remove a screw due to its lack of hold, a larger diameter screw may then be used to replace it, which is strongly advised, given the physical limitations of the vertebra itself, or the fixation is done on the basis of another system. In order for replacement screws to have good fixation they will need two points of contact, these are the two corticals. This means that the tip of the screw will emerge, thus involving the risk of damage to major blood vessels, intestines, rectum, etc., and complications in the intervention.

On the other hand, in conventional dorsolumbar and lumbosacral fixation systems hooks are used which can be screwed directly onto the rod; with the result that the position of the hook determines the pass of the rod, i.e. its direction, thus obliging the rod to be bent to shape, this in turn must have an angle of 90°, with the problems of the loads on the rod, as described above.

Through the use of a dorsolumbar and lumbosacral vertebral fixation system all of these disadvantages can be palliated or avoided. To these elements that have been improved, in comparison with existing elements, are used, such as connectors with adjustable orifices incorporating directional swivels between the screws and the rod, as well as fixation screws that are hollow expansion screws to impede them pulling out of the bone.

With this system the couplings consist of clamps with an adjustable orifice, that allow the bone fixation screw to be fitted in various positions, thus avoiding to a great extent the need to bend the rod and eliminating risks of mis-shaping, stress, etc. The above means that the rods can be smooth and unthreaded, insofar as threading leads to stress-raisers, and without the need for a lot of nuts, in such a way that good vertebral fixation can be achieved with a reduced number of parts.

On the other hand the coupling and the rod are fitted with an open swivel that allows for the orientation of one part in relation to another, the rigorous 90° of other systems is avoided, as is torsion and loads on the rod, increasing the life of the implant, eliminating surgical problems, definitively cutting down intervention time and decreasing the risks of complications.

The screws used are hollow, like plugs, so that when a pin is passed through the middle they expand at the end inserted into the bone, thus increasing their resistance to pull out, making it unnecessary to traverse the two corticals of the bone and eliminating the risk of damage to blood vessels, tendons, etc..

The hooks used for this system are joined to the coupling in the same way as the screws, i.e. the threaded tail is inserted into the adjustable orifice of the coupling, being attached behind by the locknuts, essentially using two types of hook, laminar and pedicle, depending on where they are to be fitted.

To sum up, this system helps to reduce surgical intervention time and, therefore, the exposure of the opened body to the atmosphere, and simplifying to a great extent the instruments required.

In continuation, in order to contribute to a better understanding of this descriptive report, and forming an integral part hereof, a series of figures are attached, for illustrative purposes but not limitative, representing as follows:

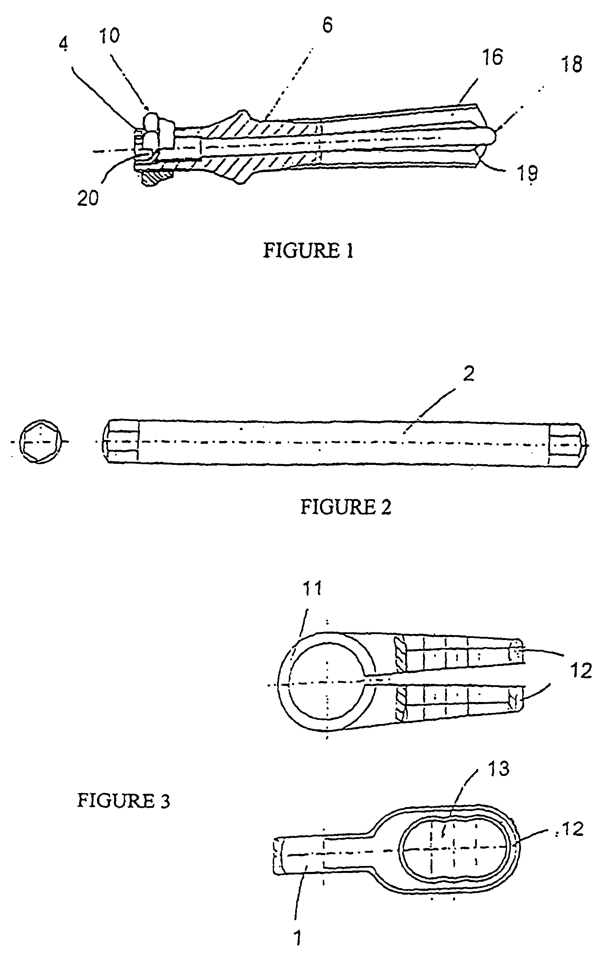

- Figure 1.-: Cross section of a practical embodiment with an expansion screw.
- Figure 2.-: Elevation view of the system rod.
- Figure 3.-: Elevation and plan view of a coupling or connector.

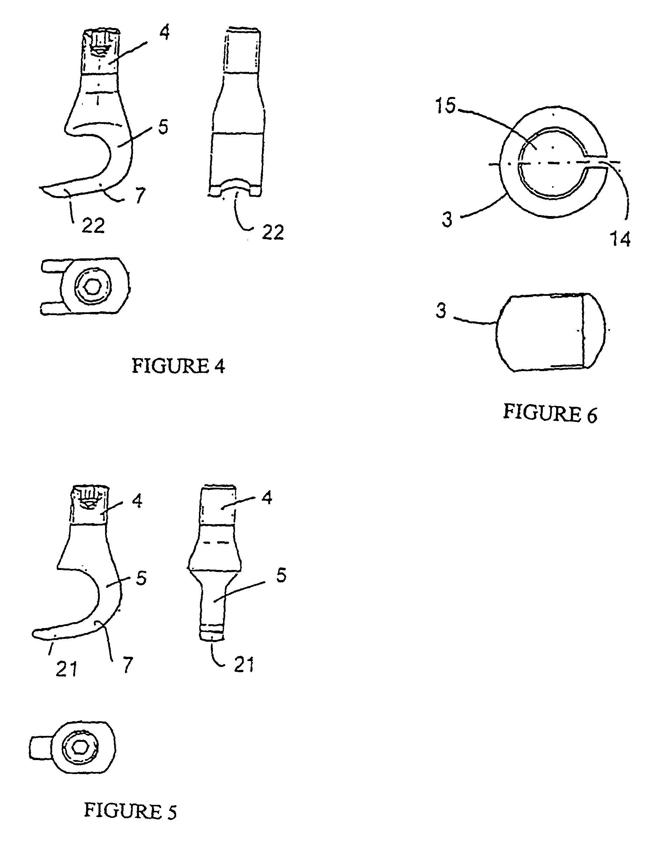

- Figure 4.-: Elevation views and profile view of a pedicle hook.
- Figure 5.-: Elevation views and profile view of a laminar hook.
- Figure 6.-: Elevation view and plan view of a multidirectional swivel with a transpedicle screw.

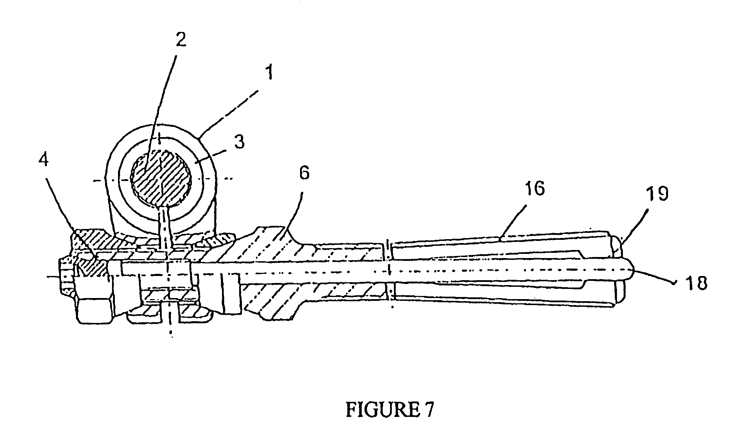

- Figure 7.-: Cross section of an assembly of the system, object of the present inven- tion.

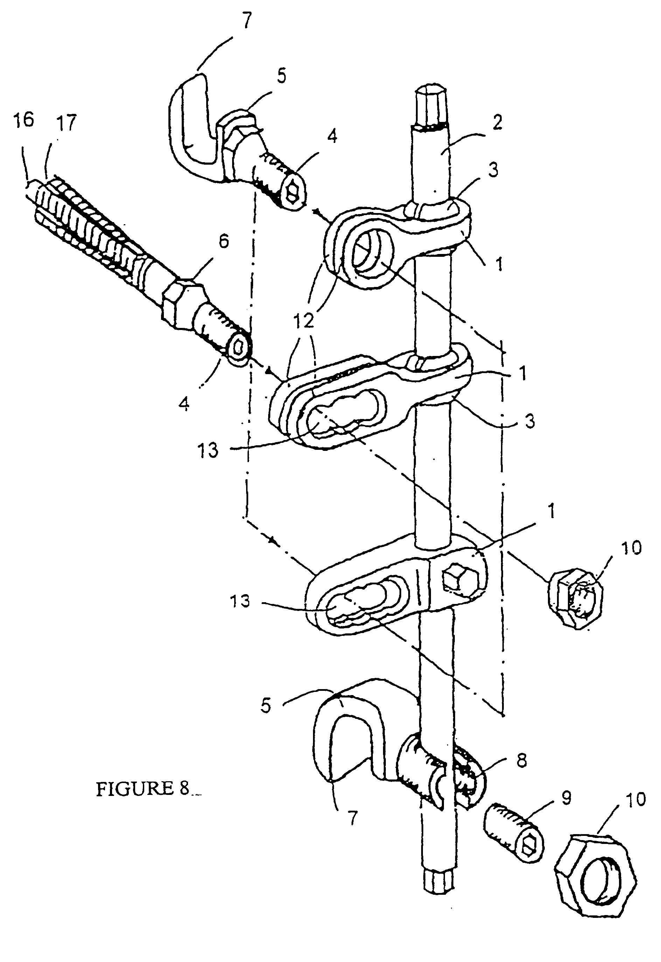

- Figure 8.-: Perspective drawing of a practical embodiment of the vertebral fixation system, object of the present invention.

As can be seen in the figures various parts are distinguished as part of the system, on the one hand the couplings or connectors (1) with their swivels (3), a rod (2), a screw (6) with a tail (4) to screw into the coupling, and also hooks (5) with their tails (4) and their respective fingers (7) to be attached to the vertebra, or this hook to be connected directly to the rod (2) by means of a split head (8), with closure using a setscrew (9) and a nut (10).

These couplings or connectors (1) essentially consist of an annular part (11), with two clamping elements (12), in such a way that they allow for a certain flexibility of the body, given that they naturally adopt an open position. On the other hand these clamps have a transverse adjustable orifice (13), an orifice that can be circular, as required, for the precise positioning of the tail (4) of the vertebral attachment element.

The annular body (11) houses the open swivel (3) in such a way that with the clamp in its natural position the swivel turns freely in its housing with three degrees of freedom, with the exterior radius of the swivel (3) slightly lower than the inside of the ring (11) of the coupling (1), both being concentric radii.

The swivel consists of a body (3) opened by a slot (14), with a passing orifice (15), through which the rod (2) of the system can pass. The exterior surface of this swivel has a rough finish so that when it is pressed onto the clamp (12) and the opening slot (14) is closed, then the contact between the exterior surface of the swivel (3) and the interior surface of the annular body (11) of the coupling (1) prevents all movement.

One of the main elements of this system is the pedicle screw (6), consisting of a tail (4) for connection to the coupling (1) and the rod (2), and a body with slots (17) that start half way along the threaded length forming lugs (16), with the body of the screw, externally threaded, hollow and smooth on the inside, allowing it to house a pin (18) which slides against an inclined plane (19), forcing it to open pressing against the sponginess of the vertebral body, in such a way that the diameter of the lower third of the expansion screw (6) increases progressively towards the end, until it reaches its maximum at a point between 20 and 30% when completely expanded.

The tail (4) of the screw (6) has an interior thread with which to pick up the head (20) of the pin (18), which is machine on the interior, like an Allen-screw, allowing it to be attached to the screw (6).

Other fixation elements that are used for the coupling of the system to the vertebra are hooks (5), fitted with a tail (4) for their upper connection to the coupling (1) and a vertebra coupling finger (7), this finger being smooth (21) in the case of laminar hooks or concave (22) for pedicle hooks.

Another of the hooks (5) used with this system is a hook with an open tail (8), inside of which the rod (2) is fitted, subsequently locked into place by a setscrew (9) on the inside and a locknut (10) on the outside.

Application and operation are simple. The system is designed for dorsolumbar and lumbosacral spinal pathologies, such as degenerative pathologies, osteoporosis, tumours, fractures, re-interventions on the sacral spine and spinal deformities (scoliosis).

The approach path is posterior, using screws (6) and hooks (5) according to the level and/or the pathology concerned.

Firstly the pedicle is located, for the use of the expansion screw (6), a bit and tapper are used to make a hole in the pedicle, then the screw (6) is inserted in the hole in the vertebra with the pin (18) inside the hollow body of the screw (6), but not fully housed. This assembly is inserted into the hole that has been made. All of the screws (6) required by the system are inserted in this way.

At the same time an assembly is put together with the open swivel (3) and the coupling (1), by means of the annular body (11). These couplings or connectors (I) are assembled on the rod (2), this is done by passing the rod through the swivel orifice (15).

Once the respective couplings (1) have been positioned on the rod, aligned with the inserted screws (6), the threaded tails (4) of the screws (6) are inserted through the adjustable orifices (13) of the coupling clamp (12), in the most adequate position for the assembly of the system. In those cases in which this coupling does not have adjustable holes, it will have to be inserted through normal circular orifices.

In this position the couplings (1) are orientated towards the tails (4) of the screws (6), to this end the multidirectional property of the open swivels (3) is used by merely turning the coupling around the rod (2), allowing for 3 degrees of freedom in this articulation.

Once all of the parts have been coupled, the nut (10) is attached and locked onto each of the tails (4) of the different screws. This nut is then made up, thus closing the clamp (12), which in turn closes the opening slot (14) of the swivel (3), thus attaching the system, given that, on the one hand, the swivel is fixed to the exterior surface of the rod (2), and on the other the coupling (1), by means of the rough contact against the interior surface of the annular body (11), eliminates any possibility of swivel (3) movement.

When this task has been concluded, the pin (18) is inserted as far as it will go, using a screwdriver, thus forcing the screw (6) to expand. To do this the screwdriver is applied to the head of the pin (20) which is housed inside the tail (4) of the screw. As the point of the pin (18) is made up it meets the inclined plane (19) of the smooth interior hollow of the screw, as it continues advancing this pin opens out the end of the screw, thanks to the slots (17) forcing the lugs (16) of the screw to expand against the sponginess of the vertebra, until it reaches the stop and the screw is completely expanded.

For those cases in which the use of hooks (5) is indicated, such as cases of the upper part of the spinal column, then the grounding of the hook (5) is worked with a suitable instrument, coupling it to the finger (7), either laminar in cases of laminar fingers (21) or gripping the pedicle through the concave shape (22) of the finger (7), depending on which hook (5) is used and the procedure.

Subsequently the coupling (1) connection is carried out in a similar way to the assembly of the expansion screws (6).

The fitting of open tail (8) hooks (5) is somewhat similar, with the rod (2) being passed through them, and with the assembly finally being closed in by a setscrew (9) on the inside and a locknut (10) on the outside.

Having sufficiently described the nature of the present invention, as well as the means for it to be put into practice, it only remains for us to add that the introduction of changes of form, both overall and in terms of the different parts that make up the invention, as well as in terms of both materials and the layout thereof, is possible, always insofar as such alterations do not substantially vary the characteristics of the invention that is distinguished in the following claims.

## Claims

1. A dorsolumbar and lumbosacral vertebral fixation system, distinguished because the system consists of one or various connectors or couplings (1), a rod (2), a transversal traction device and means of vertebral fixation, with assembly carried out by the attaching the tail (4) of the vertebral element - coupling (1) - rod (2), the first assembly stage of the system being the introduction of the fixation elements, either to the pedicle or the vertebral laminae, a second stage of the insertion of the rod (2) through the connectors (1), and a third stage in which the connectors are connected to the tails (4) of the fixation elements by means of locknuts (10).

2. The dorsolumbar and lumbosacral vertebral fixation system, as in the first claim, **characterized in that** the connectors (1) are made up of an annular body (11) and two clamp elements (12) with the insertion of an open swivel (3) inside the annular body (11).

3. The dorsolumbar and lumbosacral vertebral fixation system, as in the preceeding and in the first claim, **characterized in that**, with the clamp (12) open in its natural position, the swivel (3) turns freely in its housing, with three degrees of freedom, in a radius exterior to the swivel (3) slightly smaller than the inside of the ring (11) of the clamp, both being concentric radii.

4. The dorsolumbar and lumbosacral vertebral fixation system, as in the first and the previous claims, **characterized in that** the open clamp (12) has a transversal circular orifice into which the tail (4) of the fixation element is inserted.

5. The dorsolumbar and lumbosacral vertebral fixation system, as in the first and the previous claim, **characterized in that** the open clamp (12) has an adjustable transversal orifice (13) that allows for different positions of the tail (4) of the vertebral fixation elements.

6. The dorsolumbar and lumbosacral vertebral fixation system, as in the first and the previous claim, **characterized in that** the open swivel (3) is hollow with a circular shape (15) through which the rod (2) passes.

7. The dorsolumbar and lumbosacral vertebral fixation system, as in the first and the previous claim, **characterized in that** the exterior surface of the swivel (3) has a rough finish, which allows for better contact between surfaces when tightened.

8. The dorsolumbar and lumbosacral vertebral fixation system, as in the previous claims, **characterized in that** the screw tightness of the tail (4) of the vertebral fixation element on the clamp (12) closes the body of the clamp which, in turn, closes the opening-slot (14) of the swivel (3), thus tightening onto the previously oriented rod (2), fixing it in place.

9. The dorsolumbar and lumbosacral vertebral fixation system, as in the first claim, **characterized in that**, as a vertebral fixation element, an expansion screw (6) is used, this being a hollow pedicle screw, smooth on the inside, through which a pin (18) is passed.

10. The dorsolumbar and lumbosacral vertebral fixation system, as in the first and the previous claim, **characterized in that** the screw head has an interior thread in order to house, threaded in, the Allen-type screw of the screw head (20) of the pin (18).

11. The dorsolumbar and lumbosacral vertebral fixation system, as in the first and the ninth claim, **characterized in that** the expansion screw consists of lengthways slots (17), which start towards the middle of its threaded length, and which are opened by fully inserting the pin (18).

12. The dorsolumbar and lumbosacral vertebral fixation system, as in the first and previous claim, **characterized in that** the diameter of the lower third of the expansion screw (6), when the pin is fully inserted, progressively increases towards the end, until it reaches its maximum at the tip, between 20 and 30% when completely expanded.

13. The dorsolumbar and lumbosacral vertebral fixation system, as in the first and the previous claim, **characterized in that** the expansion screw (6) has an interior hollow conduit, with an inclined plane (19) towards the end, in such a way that when the pin is inserted (18), without making up the head (20), the tip of this pin reaches the said inclined plane (19).

14. The dorsolumbar and lumbosacral vertebral fixation system, as in the first and the previous claim, **characterized in that**, when the head (20) of the pin (18) is made up on the tail (4) of the expansion screw (6), the tip of the pin (18) opens the inclined plane (19) forcing the slots (17) of the screw (16) to open, expanding the screw against the sponginess of the vertebral body.

15. The dorsolumbar and lumbosacral vertebral fixation system, as in the first claim, **characterized in that**, as a fixation element, a laminar (21) hook (5) is used, which couples onto the vertebral lamina by means of a hook finger and is screwed to the coupling (1) at the top.

16. The dorsolumbar and lumbosacral vertebral fixation system, as in the first claim, **characterized in that**, as a vertebral fixation element, a pedicle (22) hook (5) is used, which couples onto the pedicle of the vertebra by means of a concave shape on the finger of the hook and is screwed to the coupling (I ) at the top.

17. The dorsolumbar and lumbosacral vertebral fixation system, as in the first claim, **characterized in that**, as a fixation element, a hook (5). with an open tail (8) is used, as a top connection directly to the rod (2), being closed and attached by means of a locknut (10) and a locking setscrew (9).
